# EUROPEAN PATENT APPLICATION

(11) **EP 1 690 936 A1**
(43) Date of publication of application: **16.08.2006**
(21) Application number: 04721665.0
(22) Date of filing: 18.03.2004
(51) Int. Cl.: C12N 15/09, A61K 35/28, A61K 38/00, A61K 48/00, A61P 7/06, C12N 5/06, C12N 5/10, C07K 14/47

(54) **METHOD OF ENHANCING HEMATOPOIESIS-SUPPORTING ABILITY OF IMMORTALIZED HUMAN BONE MARROW STROMA CELL BY TRANSFERRING INDIAN HEDGEHOG GENE**

(30) Priority: 31.10.2003 JP 2003373173
(71) Applicant: Renomedix Institute Inc., Ishikari-shi, Hokkai-do 0613241 (JP)
(72) Inventor: KOBUNE, Masayoshi, Sapporo-shi, Hokkaido 064-0922 (JP); HAMADA, Hirofumi, Sapporo-shi, Hokkaido 064-0959 (JP); NIITSU, Yoshiro, Sapporo-shi, Hokkaido 064-0823 (JP)
(74) Representative: Denison, Christopher Marcus
(86) International application number: PCT/JP2004/003695
(87) International publication number: WO 2005/042740

(57) **Abstract**

hTERT-stromal cells are capable of maintaining the functions of hematopoietic stem cells over the period of 4 weeks or longer; however, the rate of stem cell amplification is relatively low. Accordingly, this invention provides a method for further amplifying hematopoietic stem cells. Via introduction of Indian hedgehog (ihh) genes into the hTERT-stromal cells with the use of a retroviral vector, hTERT-stromal cells that allow overexpression of Indian hedgehog (ihh-stromal cells) are established. Use of such ihh-stromal cells can enhance the amplification of hematopoietic stem cells.

SEQUENCE LISTING

<110> Renomedix Institute Inc.

<120> Method for increasing hematopietic support ability of immortalized
human stromal cells by introducing indian hedgehog gene

<130> PH-2100-PCT

<140>
<141>

<150> JP 2003/373173
<151> 2003-10-31

<160> 2

<170> PatentIn Ver. 2.1

<210> 1
<211> 202
<212> PRT
<213> Homo sapiens

<400> 1
Met Ser Pro Ala Arg Leu Arg Pro Arg Leu His Phe Cys Leu Val Leu
  1               5                  10                  15

Leu Leu Leu Leu Val Val Pro Ala Ala Trp Gly Cys Gly Pro Gly Arg

            20                  25                  30

Val Val Gly Ser Arg Arg Arg Pro Pro Arg Lys Leu Val Pro Leu Ala
        35                  40                  45

Tyr Lys Gln Phe Ser Pro Asn Val Pro Glu Lys Thr Leu Gly Ala Ser
    50                  55                  60

Gly Arg Tyr Glu Gly Lys Ile Ala Arg Ser Ser Glu Arg Phe Lys Glu
65                  70                  75                  80

Leu Thr Pro Asn Tyr Asn Pro Asp Ile Ile Phe Lys Asp Glu Glu Asn
                85                  90                  95

Thr Gly Ala Asp Arg Leu Met Thr Gln Arg Cys Lys Asp Arg Leu Asn
            100                 105                 110

Ser Leu Ala Ile Ser Val Met Asn Gln Trp Pro Gly Val Lys Leu Arg
        115                 120                 125

Val Thr Glu Gly Trp Asp Glu Asp Gly His His Ser Glu Glu Ser Leu
    130                 135                 140

His Tyr Glu Gly Arg Ala Val Asp Ile Thr Thr Ser Asp Arg Asp Arg
145                 150                 155                 160

Asn Lys Tyr Gly Leu Leu Ala Arg Leu Ala Val Glu Ala Gly Phe Asp
                165                 170                 175

Trp Val Tyr Tyr Glu Ser Lys Ala His Val His Cys Ser Val Lys Ser
            180                 185                 190

Glu His Ser Ala Ala Ala Lys Thr Gly Gly
        195                 200

<210> 2
<211> 609
<212> DNA
<213> Homo sapiens

<400> 2
atgtctcccg cccggctccg gccccgactg cacttctgcc tggtcctgtt gctgctgctg 60
gtggtgccgg cggcatgggg ctgcgggccg ggtcgggtgg tgggcagccg ccggcgaccg 120
ccacgcaaac tcgtgccgct cgcctacaag cagttcagcc ccaatgtgcc cgagaagacc 180
ctgggcgcca gcggacgcta tgaaggcaag atcgctcgca gctccgagcg cttcaaggag 240
ctcaccccca attacaatcc agacatcatc ttcaaggacg aggagaacac aggcgccgac 300
cgcctcatga cccagcgctg caaggaccgc ctgaactcgc tggctatctc ggtgatgaac 360
cagtggcccg gtgtgaagct gcgggtgacc gagggctggg acgaggacgg ccaccactca 420
gaggagtccc tgcattatga gggccgcgcg gtggacatca ccacatcaga ccgcgaccgc 480
aataagtatg gactgctggc gcgcttggca gtggaggccg gctttgactg ggtgtattac 540
gagtcaaagg cccacgtgca ttgctccgtc aagtccgagc actcggccgc agccaagacg 600
ggcggctaa 609

## Description

### Technical Field

The present invention relates to the proliferation of hematopoietic lineage cells. More particularly, the present invention relates to the proliferation of human hematopoietic stem cells or cord-blood-derived CD34+ cells. Further, the present invention relates to a method for enhancing the hematopoiesis-supporting ability of immortalized human bone marrow stromal cells.

### Background Art

In recent years, regenerative medicine that utilizes human hematopoietic stem cells has gained attention, and the applicability thereof to regeneration of tissues, such as blood vessel, cardiac muscle, or liver tissues, as well as to bone marrow transplantation, has been suggested. However, the frequency of appearance of hematopoietic stem cells among hematopoietic cells is very low, which hinders extensive clinical applications of such cells. If a large quantity of hematopoietic stem cells could be applied to tissue regeneration, it could lead to the development of an effective therapeutic method. Over the past decade, amplification of hematopoietic stem cells *ex vivo* has been attempted to a great extent. As a result of such studies, it has been elucidated that functions of stromal cells (i.e., bone marrow stromal cells) are important in order to maintain the capacity for autonomous replication (selfrenew) and the capacity for multipotential differentiation of hematopoietic stem cells.

For example, attempts to amplify hematopoietic stem cells with the use of bone marrow stromal cells of mice, swine, or simians have been made. However, use of stromal cells obtained from different animal species may lead to contamination with foreign antigens, and thus, clinical applications thereof have not been easily realized.

It was recently reported that use of primary human stromal cells enables the amplification of human hematopoietic progenitor cells in serum-free medium. However, it was difficult to carry out long-term subculture of primary human stromal cells, and, disadvantageously, their proliferation stops at approximately the 20th generation and aging begins (Experimental Hematology 29, 174-182).

The present inventors have already attempted various techniques for immortalizing human bone marrow stromal cells in order to realize the long-term subculture of primary human stromal cells. As a result of such attempts, they have succeeded in demonstrating that primary human stromal cells could endure long-term subculture of over approximately 100 generations for 500 days or longer without tumorigenic transformation with the simple introduction of a telomerase gene (hTERT)-expressing retroviral vector. Further, they also demonstrated that coculture of hTERT-stromal cells and human cord-blood-derived CD34+ cells enables the amplification of CD34+ hematopoietic progenitor cells to about 500 times the initial level in 2 weeks, and that it enables the subsequent production of hematopoietic progenitor cells in amounts about 500 times greater than the amount of the hematopoietic cells that had invaded the stromal cells per week (WO 03/038076).

It has been known that hematopoietic stem cells appear during embryonic development and that they are rapidly amplified before birth. Some of such molecular mechanisms have been elucidated in recent years. Dyer-MA et al. reported that hematopoiesis and vascularization are induced upon reactions of the Indian hedgehog on the primitive ectoderm during embryonic development (Development, 2002, 129: 361-372). It is also reported that the Sonic hedgehog associated with neural differentiation during embryonic development enables the amplification of CD34+ hematopoietic stem cells (Mech Dev., 2001, 106: 107-117).

Members of the hedgehog gene family encode classes of secretory proteins that function as intracellular signals, and known vertebrate hedgehog genes include the Sonic, Indian, and Desert types. The hedgehog genes are evolutionarily preserved and they regulate normal growth and patterning in various animal species. In the case of vertebrates, homologues of hedgehog genes are expressed in a tissue-specific manner, and they play a key role in the morphogenesis of various organs, such as neural canals and skeletons. The active signal (transmission) of the aforementioned protein is bound to cholesterol during the self-decomposition of full-length Hh, and this modification results in the formation of a concentration gradient in the protein tissues, which is in turn associated with tissue conformation.

### Disclosure of the Invention

The hTERT-stromal cells established by the present inventors maintain hematopoiesis-supporting ability even after cryopreservation, and such cells can be established regardless of donor. This has enabled the amplification of autologous hematopoietic precursor or stem cells with the use of autologous stromal cells, when the need arises.

The hTERT-stromal cells can maintain functions of hematopoietic stem cells over a long period of 4 weeks or longer; however, the amplification rate of stem cells is found to be lower than the case where cells are derived from mice. Accordingly, it was considered that some efforts should be made in order to amplify hematopoietic stem cells.

In order to overcome such problems, the present inventors introduced Indian hedgehog (hereafter also referred to as "ihh") genes into the hTERT-stromal cells using a retroviral vector to establish hTERT-stromal cells (Indian hedgehog/hTERT-stromal cells) that allow overexpression of Indian hedgehog genes. The resulting stromal cells were subjected to coculture with human CD34+ positive cells. As a result, the present inventors discovered that such stromal cells allowed the amplification of CD34+ positive cells 3.6 times, colony-forming units mixed (CFU-mix) cells (primitive colony-forming cells) 5.5 times, and highly proliferative potential-colony forming cells (HPP-CFC) 8.9 times or more, compared with the control stromal cells. They also discovered that the proliferated blood cells would potently repopulate bone marrow cells. The present inventors completed the present invention based on such findings.

This description includes part or all of the contents as disclosed in the description and/or drawings of Japanese Patent Application No. 2003-373173, which is a priority document of the present application.

### Brief Description of the Drawings

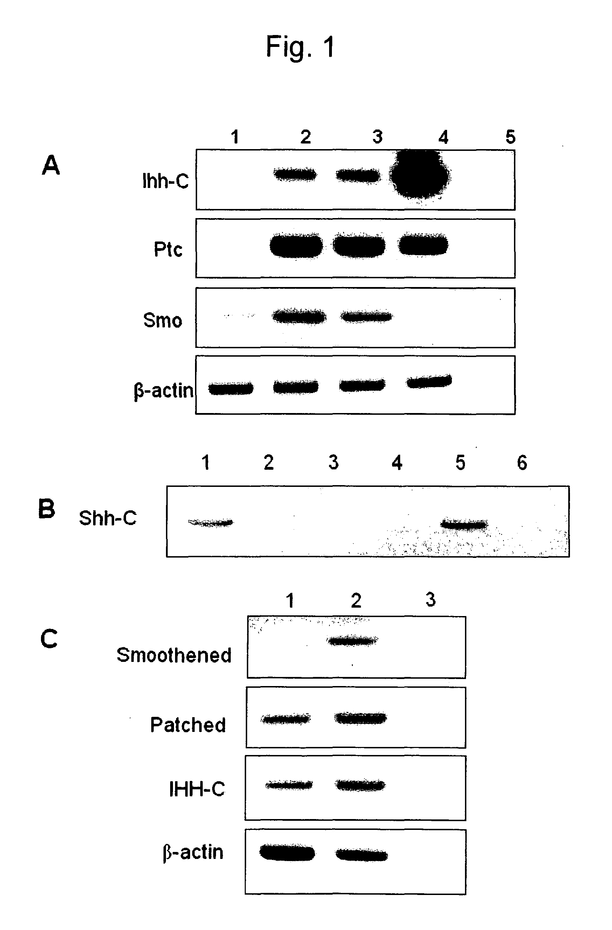

Fig. 1A shows the expression of Indian hedgehog mRNA and that of mRNA encoding the receptor molecules thereof. A primer specific to the C terminus of ihh was used (Table 1). (Lane 1: HUVEC; lane 2: primary stromal cells; 3: hTERT-stromal cells; 4: cord blood; 5: H₂O)
Fig. 1B shows the expression of Sonic hedgehog (Shh) mRNA. RT-PCR was carried out using the primer set for Shh-1 shown in Table 1. (Lane 1: HUVEC; lane 2: primary stromal cells; 3: hTERT-stromal cells; 4: cord blood; 5: thymic gland; 6: H₂O)
Fig. 1C shows the expression of the hedgehog receptor molecule mRNA in cord blood. (Lane 1: whole cord blood; lane 2: CD34+ RT+ cells; lane 3: CD34+ RT- cells (i.e., the control that was not subjected to RT reactions))

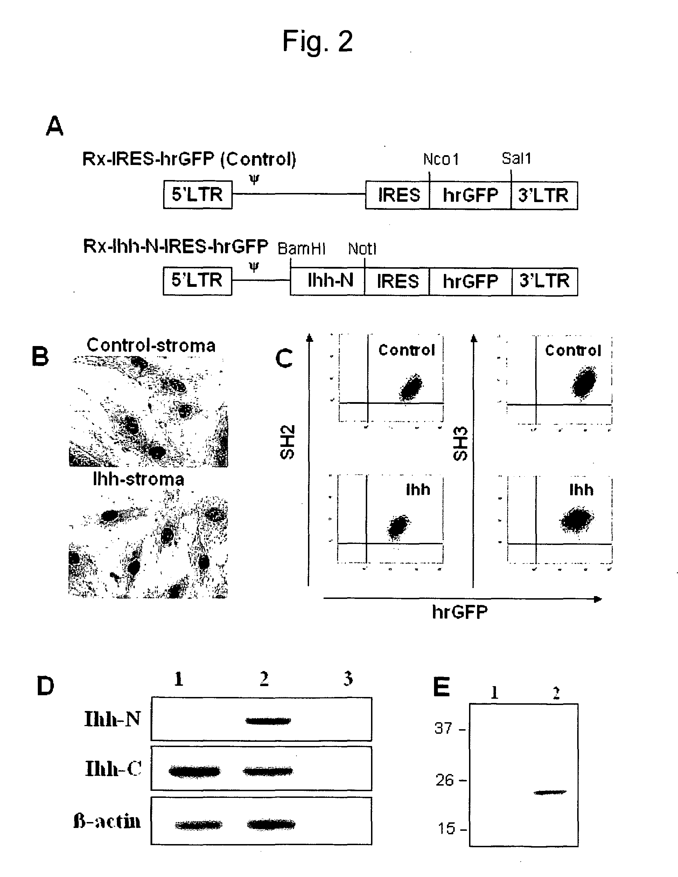

Fig. 2A shows a retroviral vector, wherein LTR represents a long terminal repeat, Ψ represents a packaging signal, hrGFP represents a humanized Renillareniformis green fluorescent protein, and IRES represents an internal ribosome entry originating from EMCV viral configuration. An empty vector was employed as a control. Fig. 2B shows the results of May-Giemsa staining of the control stromal cells into which both vectors shown in A had been introduced and of the ihh-stromal cells. Fig. 2C shows SH2, SH3, and GFP expressions in ihh-stromal cells. Fig. 2D shows the results of the analysis of ihh mRNA expression via RT-PCR specific to ihh-N and ihh-C, wherein lane 1 represents control stromal cells, lane 2 represents ihh-stromal cells, and lane 3 represents a negative control (H₂O). Fig. 2E shows the results of the immunoblot analysis of ihh-N in the supernatant of human stromal cells. Ihh-N was labeled with an anti-hedgehog 5E1 antibody. Lane 1 represents control stromal cells and lane 2 represents ihh-stromal cells.

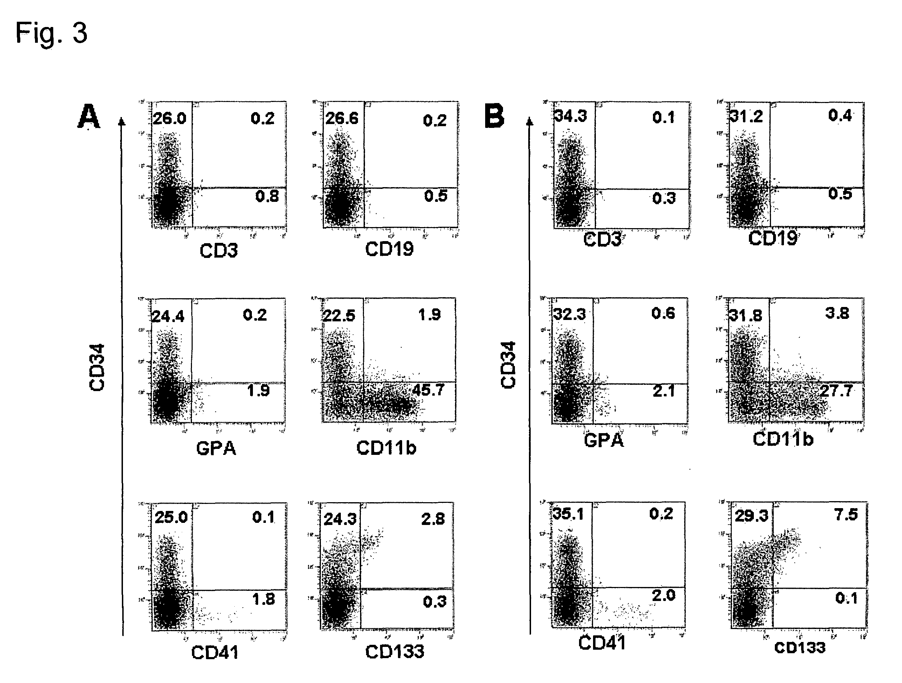

Fig. 3 shows surface antigen expression on primitive hematopoietic cells generated after the amplification of cord-blood-derived CD34+ cells in the presence of the control stromal cells (A) or in the presence of the ihh-stromal cells (B) for 2 weeks. Fig. 3 shows surface antigen expression on primitive hematopoietic cells generated after the amplification of cord-blood-derived CD34+ cells in the presence of the hTERT-stromal cells (A) or in the ihh-stromal cells (B) for 2 weeks. The X-axis represents the expression of CD34 labeled with a FITC-conjugated monoclonal antibody. The Y-axis represents the expression of CD3, CD 19, GPA, CD11b, or CD41 labeled with a PE-conjugated monoclonal antibody. Surface antigen positivity was determined in comparison with the isotype control monoclonal antibody. The data obtained from one representative experiment among 4 experiments exhibiting similar results are shown.

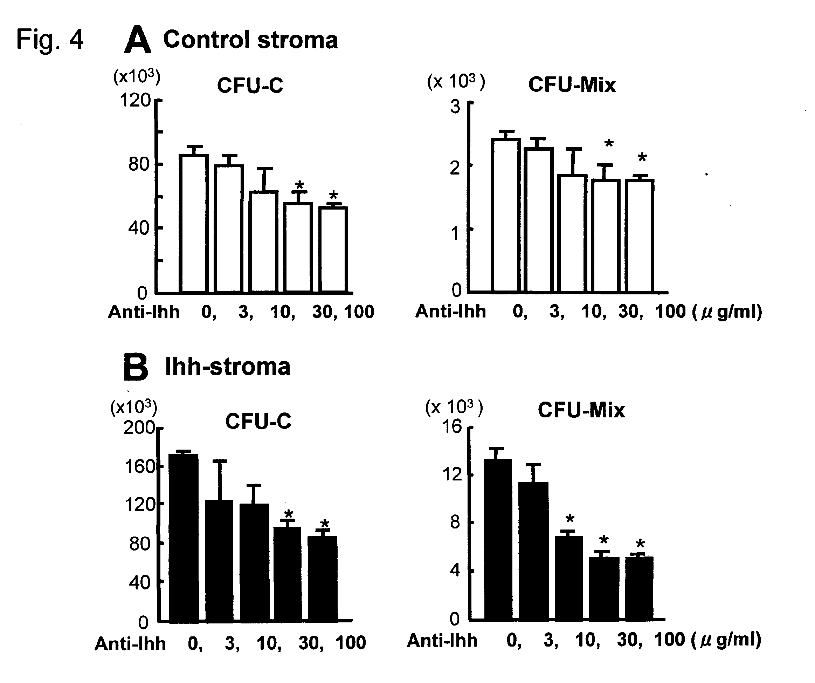

Fig. 4 shows the ihh inhibition experiment involving the 5E1 monoclonal antibody without stromal cells. The amplified hematopoietic (HPS) cells were sampled 2 weeks after the initiation of experiment and then analyzed. The horizontal axis represents a concentration of the 5E1 antibody. Fig. 4A shows the cell counts of the colony-forming units in culture (CFU-C) and CFU-mix upon coculture with the control stromal cells, and Fig. 4B shows the same upon coculture with the Ihh-stromal cells. CD34+ cell counts were determined based on the rate of CD34+ cells determined by flow cytometric analysis. The asterisks in the drawing represent the results obtained via the student's t-test on primary stromal cells with a significance level of p < 0.05.

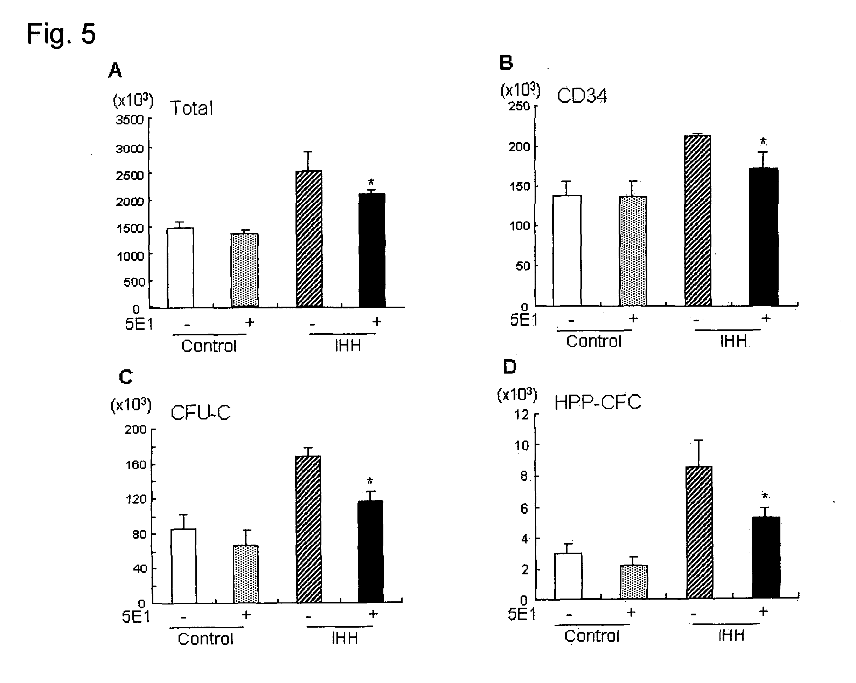

Fig. 5 shows the ihh inhibition experiment involving the 5E1 antibody upon coculture with ihh-stromal cells. The amplified hematopoietic cells (HPCs) were sampled 2 weeks after the initiation of experiment and then analyzed. The X-axis represents the duration of cell proliferation. The "+" symbol represents a group to which 5E1 has been added, and the "-" symbol represents a group to which 5E1 was not added. The Y-axis represents cell counts. (A) represents total cell counts, and (B) represents CD34+ cell counts. CD34+ cell counts were determined based on the rate of CD34+ cells determined by flow cytometric analysis. (C) represents colony-forming units in culture (CFU-C), and (D) represents HPP-CFU counts obtained via the student's t-test on primary stromal cells with a significance level of *p < 0.05.

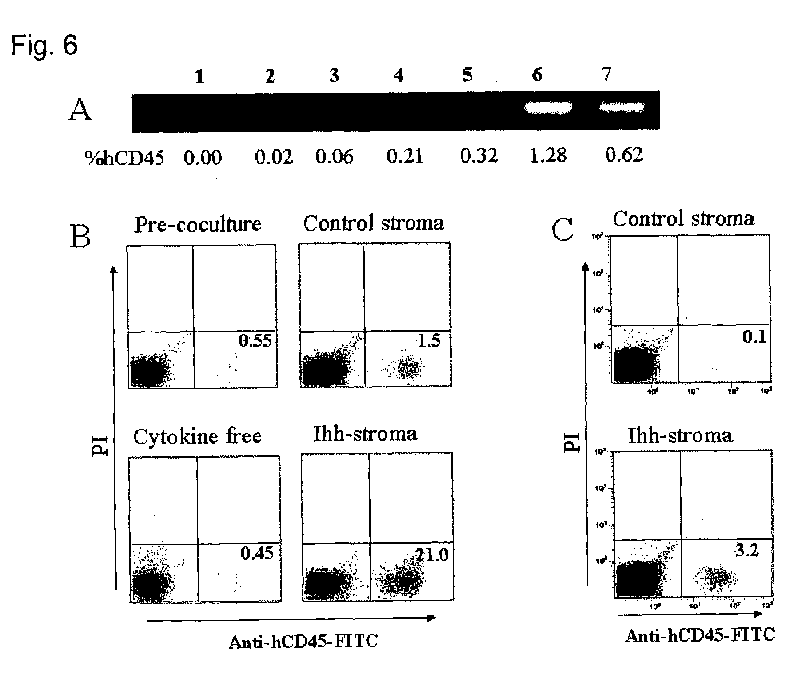

Fig. 6A shows the results of analysis of %hCD45 in human genomes and in NOD-SCID mice. A sequence that is found in the human genome upon amplification of a human ALU sequence via PCR was observed. Lanes 1, 2, and 3 each independently represent a mouse into which accessory cells had been transplanted (3 mice in total). Lanes 4, 5, 6, and 7 each independently represent a mouse into which pre-cocultured CD34+ cells had been transplanted (5 mice in total). "%hCD45" represents the rate of appearance of human CD45+ cells (HPCs) in bone marrow (BM) of the mice that had undergone transplantation. Fig. 6B shows the representative data obtained via flow cytometric analysis, with the use of an anti-human CD45 antibody, regarding mononuclear cells (MNC) in bone marrow (BM) of the NOD-SCID mouse 8 weeks after the transplantation. CD34+ cells that were not subjected to coculture were transplanted into the NOD/SCID mouse (upper left), HPC cells that were amplified without stromal cells were transplanted thereinto (lower left), control stromal cells were transplanted thereinto (upper right), and ihh-stromal cells were transplanted thereinto (lower right). Culture was conducted for 2 weeks. Fig. 6 (C) shows the representative data obtained via flow cytometric analysis regarding mononuclear cells (MNC) in bone marrow (BM) of the NOD-SCID mice 13 weeks after the transplantation. The viability (%) of human CD45+ cells amplified in the presence of the control stromal cells (upper part) or in the presence of ihh-stromal cells (lower part) for 2 weeks is represented. The Y-axis represents the results of staining with propidium iodide (PI).

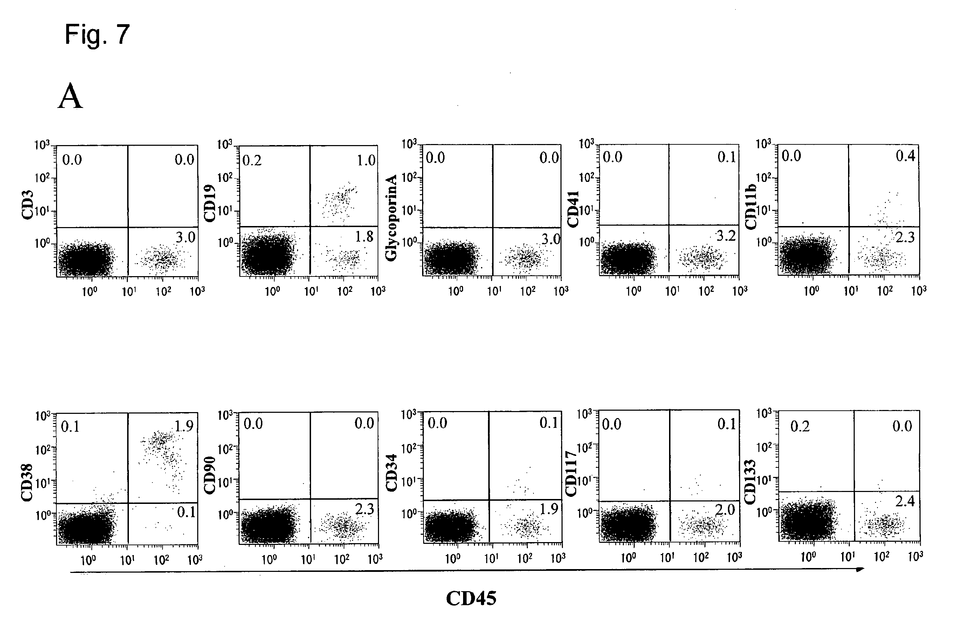

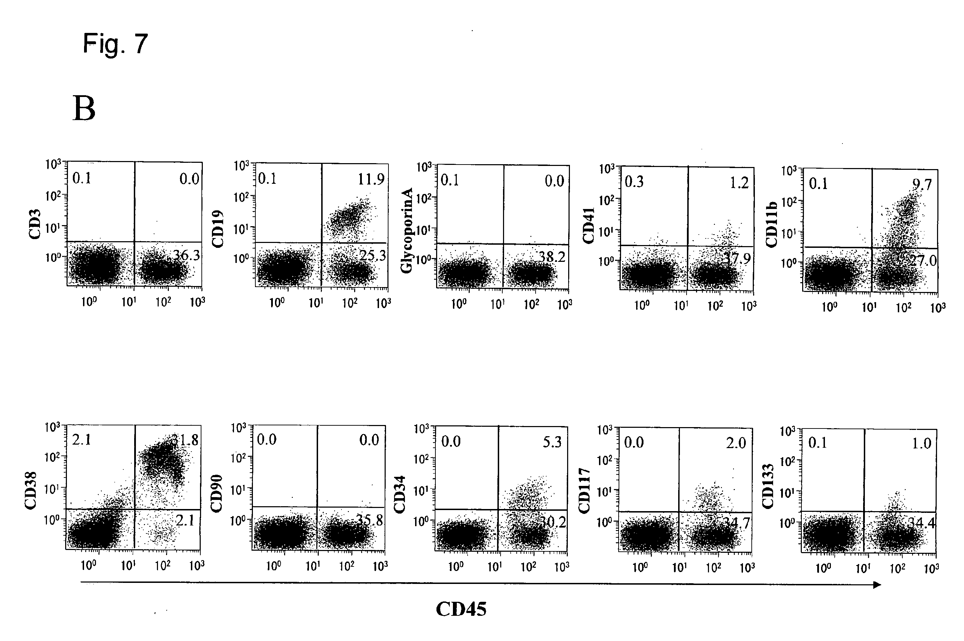

Fig. 7A shows the results of multilineage staining of HPCs. Whether or not the cells surviving in bone marrow maintained the capacity for multipotential differentiation of HPCs was determined via flow cytometry. HPCs resulting from coculture with control stromal cells were subjected to multilineage staining.
Fig. 7B shows the results of multilineage staining of HPCs. Whether or not the cells surviving in bone marrow maintained the capacity for multipotential differentiation of HPCs was determined via flow cytometry. HPCs resulting from coculture with ihh-stromal cells were subjected to multilineage staining.

### Preferred Embodiments of the Invention

The present invention provides: (1) a method for proliferating hematopoietic lineage cells using hedgehog genes, and preferably Indian hedgehog genes; (2) an agent for proliferating hematopoietic lineage cells comprising, as an active ingredient, a hematopoietic lineage cell proliferating factor of hedgehog, and preferably Indian hedgehog; (3) stromal cells that allow overexpression of hedgehog via introduction of hedgehog genes into stromal cells and a method for preparing the same (preferably, bone marrow stromal cells that allow overexpression of Indian hedgehog via introduction of Indian hedgehog genes into bone marrow stromal cells to which hTERT had been already introduced and a method for preparing the same); and (4) a method for enhancing the hematopoiesis-supporting ability of human stromal cells via coculture of the aforementioned stromal cells with hematopoietic lineage cells, such as human CD34+ cells.

The Indian hedgehog functions not only as a humoral factor but also as a membrane-bound factor via N- and C-terminal lipid modifications. Accordingly, it is considered that the Indian hedgehog enhances the capacity for supporting the differentiation of hematopoietic stem cells that proliferate by adhering to stromal cells.

The Indian hedgehog (may be hereafter abbreviated to "ihh") is synthesized as a polypeptide consisting of a total of 411 amino acid residues. The amino acid residues 1 to 27 constitute a signal peptide for protein secretion, and the amino acid residues 203 to 411 are cleaved before proteins are secreted extracellularly. Thus, ihh that functions *in vivo* is composed of the amino acid residues 28 to 202.

An example of ihh that can be employed in the present invention is (a) the Indian hedgehog as shown in SEQ ID NO: 1. Other examples thereof include (b) a polypeptide comprising at least amino acid residues 28 to 202 of the sequence as shown in SEQ ID NO: 1, (c) a polypeptide consisting of a sequence derived from the sequence as shown in SEQ ID NO: 1 by deletion, substitution, and/or addition of one to several amino acid residues and having activity of proliferating hematopoietic lineage cells, and (d) a polypeptide derived from the polypeptide composed of amino acid residues 28 to 202 of the sequence as shown in SEQ ID NO: 1 by deletion, substitution, and/or addition of one to several amino acid residues and having activity of proliferating hematopoietic lineage cells.

Examples of the Indian hedgehog genes include genes encoding the following polypeptides (a) to (d) having activity of proliferating hematopoietic lineage cells:
(a) Indian hedgehog as shown in SEQ ID NO: 1;
(b) a polypeptide comprising at least amino acid residues 28 to 202 of the sequence as shown in SEQ ID NO: 1;
(c) a polypeptide consisting of a sequence derived from the sequence as shown in SEQ ID NO: 1 by deletion, substitution, and/or addition of one to several amino acid residues and having activity of proliferating hematopoietic lineage cells; and
(d) a polypeptide derived from the polypeptide composed of amino acid residues 28 to 202 of the sequence as shown in SEQ ID NO: 1 by deletion, substitution, and/or addition of one to several amino acid residues and having activity of proliferating hematopoietic lineage cells.

More specifically, the Indian hedgehog genes comprising the following DNAs:
(a) DNA as shown in SEQ ID NO: 2;
(b) DNA comprising at least nucleotides 81 to 606 of the DNA sequence as shown in SEQ ID NO: 2; and
(c) DNA hybridizing under stringent conditions to DNA (a) or (b) and encoding a protein having Indian hedgehog activity.

Under stringent conditions, for example, the sodium concentration is 20 mM to 100 mM and the temperature is between 42°C and 55°C (Molecular Cloning, Sambrook et al. (ed), 1989, Cold Spring Harbor Lab. Press, New York).

The activity of Indian hedgehog for proliferating hematopoietic lineage cells can be determined based on the amplification or amplification rate of hematopoietic cells (HPC) in the presence of ihh-stromal cells, as described in, for example, Example 5 of the present invention.

Genes encoding a polypeptide having Indian hedgehog activity can be expressed in stromal cells. Also, Indian hedgehog can be lysed in an adequate buffer and the resultant can be directly added to a culture product of hematopoietic lineage cells and stromal cells.

Mammalian stromal cells are preferable, and human stromal cells are particularly preferable. Stromal cells include bone-marrow-derived primary stromal cells, subcultured stromal cells, and genetically engineered stromal cells. Preferably, human bone marrow stromal cells into which the hTERT genes have been introduced (hereafter referred to as "hTERT-stromal cells," disclosed in WO 03/038076) can be used. The hTERT sequence is described in, for example, Science 277, pp. 955-959.

The hTERT-stromal cells can be prepared in the following manner, for example.

The hTERT EcoRV-SalI fragment obtained via PCR from pCI-Neo-hTERT-HA is cloned into pBABE-hygro to prepare pBABE-hygro-hTERT (Proc. Natl. Acad. Sci. U.S.A., vol. 95, pp. 14723-14728). Subsequently, the Bosc23-packaging cells (Proc. Natl. Acad. Sci. U.S.A., 90, 8392-8396, 1993) are used to prepare the ΨCRIP packaging cells (Proc. Natl. Acad. Sci. U.S.A., 90, 3539-3543, 1993). The above vector is allowed to proliferate in a retrovirus-producing cell (ψCRIP/P131).

On the day before the infection, stromal cells are sowed again and cultured using a ψCRIP/P131 medium that produces retroviruses. Subsequently, the recombinant retroviral vector produced in the supernatant is allowed to infect stromal cells. The culture supernatant is replaced with fresh medium 4 hours later and cultured for an additional 2 days. Thereafter, pBABE-hygro-hTERT is subjected to drug selection in 100 µg/ml hygromycin for 5 days to prepare hTERT-stromal cells.

The Indian hedgehog genes to be introduced into stromal cells are preferably Indian hedgehog genes derived from humans. Specifically, a gene consisting of the sequence as shown in SEQ ID NO: 2 can be used.

Indian hedgehog genes can be introduced into stromal cells via various techniques for gene introduction, although lipofection is inadequate. Preferably, various types of vectors can be used, and examples of preferable vectors include retroviral, adenoviral, and adeno-associated viral vectors. Use of a retroviral vector is particularly preferable. An example of a preferable retroviral vector is pRx-IRES-neo (Hum Gene Ther., 1998, 9, 1983-1993).

The bone marrow stromal cells into which hTERT had been introduced and the bone marrow stromal cells into which Indian hedgehog genes had been introduced (occasionally referred to as "ihh-stromal cells") may be further subjected to coculture with hematopoietic lineage cells to amplify hematopoietic lineage cells.

Hematopoietic lineage cells include cord-blood-derived CD34+ cells (occasionally abbreviated to "CB CD34+"), hematopoietic cells (e.g., hematopoietic cells (HPCs) separated upon coculture of CB CD34+ cells and stromal cells), hematopoietic stem cells (HSC, e.g., human hematopoietic stem cells), hematopoietic progenitor cells, T-lymphocytes, and B-lymphocytes.

Coculture of hematopoietic lineage cells and the stromal cells of the present invention can be carried out in the presence of cytokines. Examples of such cytokines include soluble SCF (kit ligand), flt3 ligand, and thrombopoietin (TPO). More specifically, 50 ng/ml of TPO, 50 ng/ml of FL, and 10 ng/ml of SCF can be present. Coculture can be continued for 2 to 4 weeks, for example.

### [Reference Example 1]

Expression of hedgehog-associated genes and of cytokine genes in primary stromal cells and in mesenchymal stem cells

It is known that the Indian hedgehog (ihh) is secreted from cartilage cells and it plays a key role in epiphysis formation as well as in hematopoiesis at the embryonic yolk sac. In recent years, cartilage cells have been found to be differentiated from bone marrow stromal stem cells. Based on such finding, the present inventors assumed that ihh was expressed in bone marrow stromal cells and screened for the expression of hedgehog and its receptor molecules in mesenchymal stem cells, stromal cells, hematopoietic cells (cord blood), and human umbilical vein endothelial cells (HUVEC) via RT-PCR.

More specifically, total RNA was purified with the Qiagen RNeasy kit (Qiagen), and the RT reaction was carried out using SuperScript™ II reverse transcriptase (Invitrogen, Tokyo, Japan). PCR was carried out using the AccuTaq LA DNA polymerase mix (Sigma). Amplification was carried out via a cycle of denaturation at 94°C for 3 minutes, denaturation at 94°C for 30 seconds, annealing at 56°C for 30 seconds, and extension at 72°C for 60 seconds, and this cycle was repeated 35 times. Table 1 shows the primers used. The PCR product was separated via electrophoresis on 2% agarose gel, stained with ethidium bromide, and then visualized.

| Table 1: Primers used | | |
|---|---|---|
| | Sense primers | Antisense primers |
| Ihh-C | 5'CTTCCGGGCCACATTTGCCAGCCA-3' | 5'-GAGACGFCCCCAGGCGGTAGAGCA-3' |
| Ihh-N | 5'-TGCGGGCCGGGTCGGGTGGTG-3' | 5'-GCCGCCCGTCTTGGCTGC-3' |
| Shh-1 | 5'-TACAACCCCGACATCATATTTAAGG-3' | 5'-CGTCTCGATCACGTAGAAGACCTTC-3' |
| Shh-2 | 5'-CACCTGGAGCGGTTAGGGCTACTCT-3' | 5'-GCCCAGGGCACCATTCTCATCAAC-3' |
| Ptc1 | 5'-CTGTTGGCATAGGAGTGGAGTTCACC-3' | 5'-CTGCTGGGCCTCGTAGTGCCGAAGC-3' |
| Smo | 5'-CAGAACATCAAGTTCAACAGTTCAGGC-3' | 5'-ATAGGTGAGGACCACAAACCAAACCACACC-3' |
| Ang1 | 5'-GGTCACACTGGGACAGCA-3' | 5'-CGTAAGGAGTAACTGGGC-3' |
| BMP4 | 5'-GCCAAGCGTAGCCCTAAGCATCACTCA-3' | 5'-CAATGGCATGGTTGGTTGAGTTGAGGT-3' |
| β-actin | 5'-GCTCGTCGTCGACAACGGCTC-3' | 5'-CAAACATGATCTGGGTCATCTTCTC-3' |

### (Results)

As a result, ihh mRNA expression was found to be detected in bone marrow mesenchymal stem cells and in stromal cells. Interestingly, the level of ihh expression was high in a blood cell, i.e., cord blood. Further, mRNA expression of an ihh receptor molecule, i.e., patched (PTC), and that of an ihh signal transmission molecule, i.e., smoothened (SMO), were observed both in mesenchymal stem cells and in stromal cells (Fig. 1A). This was consistent with the report made by Bhardwaj et al. (Nat. Immunol., 2001, 2: 172-180).

The expression of Sonic hedgehog (SHH), which is reported to amplify hematopoietic stem cells, was also examined. Although mRNA expression was observed in human umbilical vein endothelial cells (HUVEC) and in thymic tissues (positive controls), the expression level in bone marrow stromal cells was lower than the level of detection sensitivity (Fig. 1B).

Accordingly, the present inventors assumed that ihh plays some roles in the bone marrow microenvironment. The present inventors further examined mRNA expression of the ihh receptor molecule in CD34+ cells. As a result, they observed expression of SMO, which is an ihh signal transmission molecule, in a fraction of CD34+ cells (purity: 95%), which are undifferentiated hematopoietic cells (Fig. 1C). This indicates that ihh may influence undifferentiated hematopoietic cells.

### [Reference Example 2]

### Preparation of anti-hedgehog neutralizing antibody 5E1

5E1 hybridoma cells were purchased from the Iowa Developmental Studies Hybridoma Bank. Hybridoma cells were cultured in 10% FBS-containing IMDM, 2 mM glutamine, and 1% P/S (50 U/ml penicillin, 50 mg/ml streptomycin). Pristane (1 ml) was administered intraperitoneally to the Balb/C mice a week prior to the hybridoma inoculation. Hybridoma cells at the logarithmic growth phase were washed twice with PBS sterilized in an autoclave, and a solution containing such cells in amounts of 2.5 x 10⁶ cells per ml thereof was prepared. The cells (5 x 10⁶ cells) were administered intraperitoneally with the use of a 22G needle. Thereafter, the mice were left untreated for 10 days until ascites fluid was generated. The monoclonal antibody 5E1 (IgG1) was purified from ascites fluid using a protein G column (ImmunoPure®, PIERCE). The antibody was washed with a buffer of pH 5.0 (ImmunoPure®, PIERCE) and then subjected to elution with a buffer of pH 2.8 (ImmunoPure®, PIERCE). Immediately thereafter, the antibody was neutralized with the use of 1M phosphate buffer (pH 7.5). The amount of IgG (mg/ml) was determined by the equation A280/14 x 10.

### [Example 1] Preparation of hTERT-stromal cells into which Indian hedgehog genes have been introduced (hereafter referred to as "ihh-stromal cells")

### 1. Culture of human bone marrow stromal cells

Human bone marrow cells were obtained via puncture of the posterior iliac crests of healthy adult volunteers after an informed consent process. Bone marrow mononuclear cells (MNCs) were placed in a 150-cm² plastic dish and then cultured overnight. Nonadherent cells were removed by washing and adherent cells were then used as primary bone marrow stromal cells. The hTERT-stromal cells were prepared in accordance with the method described in WO 03/038076. The transgenic stromal cell line was established with the use of the hTERT-stromal cells. Such human stromal cells were cultured in LTC medium comprising Minimal essential medium-α, 12.5% horse serum (Gibco BRL, Rockville, MD, U.S.A.), 12.5% fetal bovine serum (Gibco BRL), 1 x 10⁻⁶ M hydrocortisone (Sigma Chemical Corp., St. Louis, MO, U.S.A.), and 1 x 10⁻⁴ β-mercaptoethanol (Sigma Chemical Corp) in the presence of 5% CO₂ at 37°C. Mesenchymal stem cells that had adhered to the plastic dish were cultured in Dulbecco's modified essential medium (DMEM) supplemented with immobilized fetal bovine serum (Gibco BRL, Rockville, MD, U.S.A.).

### 2. Retroviral vector and gene introduction

In order to prepare a bicistronic retroviral vector, i.e., pRx-IRES-hrGFP, the neomycin-resistant gene of pRx-IRES-neo was substituted with the humanized Renillareniformis green fluorescent protein (hrGFP) derived from the phrGFP-C plasmid (Stratagene, La Jolla, CA) (up to 717 bp). The resultant was transformed into DH5α competent cells (Life Technologies, Grand Island, NY), and the colonies including pRx-IRES-hrGFP were then obtained by subjecting them to mini-preparation (Bio Rad, Hercules, CA), followed by digestion with restriction enzymes.

It is reported that the N-terminus of Indian hedgehog (ihh) exhibits its all activity, and the C-terminal thereof does not have any activity. cDNA of the ihh N-terminal (ihh-N, 1-202) was synthesized via reverse transcription using cord-blood-derived RNA as a template and oligo-dT primers. Polymerase chain reactions (PCR) were carried out using the following primers: 5'-AGGATCCACCATGTCTCCCGCCCGGCTCCGGCCCCGACTGCACTTC-3' and 5'-GAGCGGCCGCTTAGCCGCCCGTCTTGGCTGCGGCCGAGTG-3'.

The *Bam*HI site was incorporated into a sense primer and the *Not*I site was incorporated into an antisense primer. After agarose gel electrophoresis, the PCR product was purified and then ligated into a cloning vector, pGEM-T easy (Life Technologies, Grand Island, NY). After DH5α was transformed, the colonies containing ihh cDNA were identified via digestion with restriction enzymes. The DNA sequence was determined, and it was confirmed that the resulting ihh-N was acceptable.

In order to prepare a pRx-ihh-IRES-hrGFP retroviral vector, cDNA at the ihh-N terminus was cleaved from pGEMT easy via double digestion with *Bam*HI and *Not*I restriction enzymes (New England Biolabs, Beverly, MA), followed by purification. The resultant was ligated to the *Bam*HI and *Not*I sites of pRx-IRES-hrGFP. After DH5α was transformed, the colonies that contain pRx-ihh-IRES-hrGFP were identified via digestion with endonucleases (Fig. 2A).

### 3. Preparation of recombinant retroviral vector-producing cells

(1) The Phoenix-AMPHO cells were sowed on a 10-cm dish 18 to 24 hours before transfection at a cell density of 5.5 x 10⁶.
(2) OPTI-MEM (800 µl, Gibco/BRL) was gently added to 15 µg of the 2 aforementioned types of retroviral vectors pRx-IRES-hrGFP and pRx-ihh-IRES-hrGFP, and the mixture was stirred to prepare a solution A.
(3) OPTI-MEM (750 µl) was placed into a sterilized tube, 50 µl of Lipofectamine (2 mg/ml, Gibco/BRL) was added thereto, and the contents of the tube were slowly mixed to prepare a solution B.
(4) The solution A was gently mixed with the solution B to prepare a solution C, and the resultant was allowed to stand at room temperature for 30 to 45 minutes.
(5) The Phoenix-AMPHO cells were washed once in a medium at 37°C from which antibiotics and FBS had been removed.
(6) The solution C (1.6 ml) was gently added to the Phoenix-AMPHO cells.
(7) OPTI-MEM (2.4 ml) was further added.
(8) Incubation was carried out in the presence of 5% CO₂ for 5 hours.
(9) DMEM (4 ml) containing 20% fetal bovine serum was added and incubation was carried out overnight.
(10) The medium for the Phoenix-AMPHO cells was filtered through a 0.45-µm syringe filter 24 hours later, and the medium for the Phoenix-AMPHO cells was replaced with 5 ml of a filtered medium. Simultaneously, polybrene (hexadimethrine bromide, Sigma H-9268) was added to a concentration of 8 µg/ml, and the resultant was cryopreserved at -80°C.

### 4. Transfection into bone marrow stromal cells

Subsequently, the 2 aforementioned types of retroviral vectors were transfected into bone marrow stromal cells. (The hTERT-stromal cells into which pRx-ihh-IRES-hrGFP had been introduced are the ihh-stromal cells prepared in Example 1.)

At the outset, the hTERT-stromal cells were sowed again at a cell density of 5 x 10⁴ cells/10-cm-dish on the day before the transfection, the cryopreserved supernatant was thawed in an incubator at 37°C on the day of transfection, and recombinant retroviral vectors were allowed to infect bone marrow stromal cells. The medium was replaced with fresh medium 4 hours later and the culture supernatant was cultured for an additional 2 days (Fig. 2B).

With the use of the FACSCalibur (Becton Dickinson, Mountain View, CA), 50,000 GFP+ stromal cells were sorted. Purities of the sorted GFP+ cells were 96.7% and 93.3% in the control stromal cells (and the hTERT-stromal cells into which pRx-IRES-hrGFP had been introduced are referred to as "control stromal cells" in the following examples) and in the ihh-stromal cells, respectively.

### [Example 2] Confirmation of surface antigen and gene expressions of stromal cells to which ihh has been introduced

(1) Whether or not stromal antigen expression was maintained in the ihh-expressing cells obtained in Example 1 (ref), the expression of SH2 (CD 105, endoglin) and SH3 (CD73), and the rate of GFP+ cells, were examined.

Stromal cell surface antigens were analyzed via flow cytometry. The cells were washed twice with PBS containing 0.1% bovine serum albumin (BSA), and 5 x 10⁵ cells were labeled with anti-CD73 (SH3) (Alexis Biochemicals, San Diego, CA, U.S.A.) or anti-CD105 (SH2) (Immunotech, Marseille, France) antibodies at 4°C for 30 minutes. R-phycoerythrin (PRE) F(ab')₂ was used as a secondary antibody. The cells were analyzed using the EPICS XL flowcytometer. The dead cells were gated by forward scattering and side scattering for removal.

The rate of GFP expression was 90% or higher both in control and ihh-stromal cells, and the expressions of stromal antigens, such as SH2 and SH3, were maintained in GFP+ cells (Fig. 2C).

### [Example 3] Confirmation of gene expression in ihh-stromal cells

(1) Expression of the hedgehog gene in the hTERT-stromal cells was analyzed via RT-PCR. Total RNA was purified with the Qiagen RNeasy kit (Qiagen), and the RT reaction was carried out using SuperScript™ II reverse transcriptase (Invitrogen, Tokyo, Japan). PCR was carried out using the AccuTaq LA DNA polymerase mix (Sigma). Amplification was carried out via a cycle of denaturation at 94°C for 3 minutes, denaturation at 94°C for 30 seconds, annealing at 56°C for 30 seconds, and extension at 72°C for 60 seconds, and this cycle was repeated 35 times. Table 1 shows the primers used. The PCR product was separated via electrophoresis on 2% agarose gel, stained with ethidium bromide, and then visualized.
(2) Expression of ihh-N proteins in the ihh-stromal cells prepared in Example 1 was analyzed via immunoblotting. The culture supernatant (20 µl) of the ihh-stromal cells was separated via electrophoresis in 4% to 25% gradient SDS-polyacrylamide gels, and the resultant was transferred onto a PVDF membrane using the Semi-Dry Trans Blot System (Bio-Rad). The hedgehog proteins were labeled with anti-ihh antibodies (SE1, Hybridoma Bank, Iowa). The labeled proteins were detected by ECL (Amersham Pharmacia).

### (Results)

RT-PCR was carried out using primers that detect the N-terminal side of ihh. The level of ihh-N detected was lower than the level of detection sensitivity in the control stromal cells (where the C-terminal was detectable); however, ihh-N was easily detected in the ihh-stromal cells (Fig. 2D).

Subsequently, secretion of ihh-N to the culture supernatant of stromal cells was analyzed via Western blotting. The stromal supernatant was obtained via washing of the cells three times with PBS, replacement with X-Vivo10 serum-free medium, and culture for 72 hours. The 5E1 hedgehog monoclonal antibody was used. The level of ihh-N detected was lower than the level of detection sensitivity in the supernatant of the control stromal cells; however, ihh was detected in the supernatant of ihh-stromal cells (Fig. 2E).

### [Example 4] Coculture of cord-blood-derived CD34+ cells and stromal cells

The control stromal cells and the ihh-stromal cells prepared in Example 1 (40,000 cells) were placed into a 25-cm² flask. When the cells reached a subconfluent state, the cells were washed three times with PBS to remove serum contained in the stromal medium. X-Vivo10 with TPO (50 ng/ml), FL (50 ng/ml), and SCF (10 ng/ml) were added, and 5 x 10³ cord-blood-derived CD34+ cells, which were introduced into X-VIVO10 containing TPO (50 ng/ml), FL (50 ng/ml), and SCF (10 ng/ml) in advance, were further added. Thereby the stromal cells into which the genes had been introduced with the aid of a vector were cocultured in the presence of cord-blood-derived CD34+ cells for 2 weeks. The culture supernatant was recovered, and 2 x 10³ cells were used for colony assays. The colony counts were determined 2 weeks later. At the time of coculture, 500 µg of 5E1 was added to the coculture system every 3 days in order to neutralize the hedgehog. The hematopoietic cells amplified on the stromal cells were recovered and then subjected to flow cytometric analysis and colony assays. Colony assays were carried out using the MethoCult GF H4434V (Stem Cell Technologies, Vancouver, Canada), and the colony count was determined under a microscope after culture for 14 days.

### (Results)

The cells resulting from coculture with stromal cells were analyzed via flow cytometry. The rate of CD34+ cells amplified in the presence of the control stromal cells was between 22% and 27%, and the rate of CD34+ cells amplified in the presence of the stromal cells in which the expression level of ihh was high was maintained at a high level of 31% to 35%. The differentiation antigens of the amplified hematopoietic cells were examined. As a result, no difference was observed in the rates of expression of lymphocyte antigens such as CD3 or CD 19 or in expression of erythroblast and megakaryocyte antigens such as GPA or CD41. In the case of the CD11b granulocytic antigen, the percentage of positive hematopoietic cells amplified in the control stromal cells was as high as about 45%. When the cells were amplified in the stromal cells in which the expression level of ihh was high, the percentage of positive hematopoietic cells amplified therein was lower than 30%. This indicates that the differentiation of cells into granulocytes was suppressed in the ihh-stromal cells compared with the control stromal cells, and fractions of undifferentiated CD34+ cells could be maintained (Fig. 3).

Colony assays were then carried out to determine whether or not the undifferentiated hematopoietic cells were supported (Table 2). The total hematopoietic cell counts, the total CD34 cell counts, the colony-forming unit in culture (CFU-C) counts, colony-forming units mixed cell (CFU-mix, primitive colony-forming cells) counts, and the HPP-CFC cell counts obtained via amplification in the presence of the ihh-stromal cells were significantly larger than the counts of those cells amplified in the presence of the control stromal cells. As is apparent from the difference between the ihh-stromal cells and the control stromal cells, such difference was significant in hematopoietic progenitor cells, such as CFU-mix or HPP-CFC, which are capable of forming more primitive colonies.

**Table 2: Amplification of cord-blood-derived CD34+ cells (CB CD34+) ex vivo for 2 weeks**

| | Without stromal cells | Control stromal cells | Ihh-stromal cells |
|---|---|---|---|
| Total cell counts | 76 ± 36 | 490 ± 38 | 635 ± 30** |
| CD34+ cell counts | 5 ± 2 | 26 ± 2 | 95 ± 6** |
| CFC | 2 ± 1 | 26 ± 2 | 59 ± 3** |
| CFU-mix | 8 ± 7 | 63 ± 37 | 349 ± 116* |
| HPP-CFC | 12 ± 11 | 200 ± 175 | 1784 ± 1555* |

| | | | |
|---|---|---|---|
| Numerical values represent the multiples of cells amplified in relation to the initial cell counts. | | | |
| For Ihh-stroma vs. control stroma. * P < 0.05; ** P < 0.01 | | | |
| The results are represented in terms of mean ± standard deviation (n = 8). | | | |
| CFC: Colony-forming cells | | | |
| CFU-mix: Primitive colony-forming cells | | | |
| HPP-CFC: Highly proliferative potential-colony forming cells | | | |

### [Example 5] Inhibition of ihh by anti-ihh antibody

(1) A neutralization experiment was carried out using antibodies in order to examine whether or not the hematopoietic cell amplification in the ihh-stromal cells observed in Example 4 was directly mediated by ihh or indirectly mediated thereby through a hedgehog receptor, which is expressed on stromal cells.

Cord-blood (CB)-derived CD34+ cells were cultured with the ihh-stromal cells or without stromal cells (i.e., the control) in the presence of cytokines (50 ng/ml of TPO, 50 ng/ml of FL, and 10 ng/ml of SCF; cytokines of the same concentration were hereafter employed) and with or without the addition of 100 µg/ml 5E1 antibodies every 3 days. 1x PBS was added to the culture dish containing the control cells.

### (Results)

Fig. 4 shows the results. The amplified hematopoietic (HPS) cells were sampled in 2 weeks and then analyzed. The horizontal axis represents a concentration of the 5E1 antibody. Fig. 4A shows the cell counts of colony-forming units in culture (CFU-C) and CFU-mix when cocultured with the control stromal cells, and Fig. 4B shows those when cocultured with the Ihh-stromal cells. The CD34+ cell counts were determined based on the rate of CD34+ cells determined via flow cytometric analysis. The "*" symbol in the figure indicates the results obtained via the student's t-test on primary stromal cells with a significance level of p < 0.05.

Hematopoietic cell amplification in the control stromal cells and in the ihh-stromal cells was significantly suppressed by neutralizing antibodies. This indicates that ihh plays a key role in hematopoietic cell amplification mediated by stromal cells and that ihh is directly associated with enhancement of the hematopoiesis-supporting ability of ihh-stromal cells (Fig. 4).

### [Example 6]

### (1) Transplantation into NOD/SCID mice

As the targets of transplantation, 6- to 10-week-old NOD/SCID-beta2m-/- mice, which were obtained from the parent mice obtained from L. Shultz (Jackson Laboratory, Bar Harbor, ME, U.S.A.) were employed. In the presence of TPO, SCF, and FL, a layer of control stromal cells or ihh-stromal cells was cocultured with CB CD34+ cells for 2 weeks. Thereafter, the hematopoietic cells (HPCs) that had proliferated on the upper side of the layer of stromal cells were recovered. The contamination percentage of stromal cells in the hematopoietic cells was observed to be 0.01% or lower under a microscope. Stromal cells can be easily distinguished from hematopoietic cells based on the size and morphological features of cells under a microscope.
(i) CB CD34+ cells (cells before coculture with the control or Ihh-stromal cells), CB CD34+ cells cultured alone for 2 weeks (i.e., the cells obtained by culturing in the absence of stromal cells and in the presence of cytokines, such as 50 ng/ml of TPO, 50 ng/ml of FL, and 10 ng/ml of SCF), or primitive hematopoietic progenitor cells (HPCs) proliferated via coculture of CB CD34+ cells with the control or ihh-stromal cells for 2 weeks were injected into a mouse through a lateral caudal vein, which had been irradiated with 400 cGy.

The peripheral blood mononuclear cells obtained from healthy volunteers (5 x 10⁶ cells) irradiated with 1,500 cGY were transplanted to mice as accessory cells simultaneously with or independently from HPCs.

Subsequently, whether or not the amplified hematopoietic cells were viable in bone marrow and capable of repopulating bone marrow were examined.

### (Results)

The chimerisms of human blood cells in NOD/SCID mice after transplantation were analyzed via flow cytometry using anti-CD45 antibodies. As shown in Table 3, the hematopoietic cells amplified on the ihh-stromal cells for 2 weeks exhibited viability approximately 30 times higher than in a case where amplification took place in the control stromal cells. In the past, the present inventors reported that human stromal cells could assist the cells to maintain the capacity for repopulating hematopoietic cells (SRC) via simultaneous transplantation of cells that had transmigrated below the stromal cells and cells that had been amplified on the stromal cells. When clinical applications thereof were taken into consideration, however, immortalized stromal cells were preferably eliminated, and such removal has been carried out by allowing such cells to adhere to the plastic dish, although complete removal was difficult. In the present invention, the cells amplified on the ihh-stromal cells were found to be capable of sufficiently repopulating hematopoietic cells. Accordingly, it is considered that contamination by stromal cells could be relatively easily prevented via, for example, insertion of a filter between stromal cells and hematopoietic cells.

**Table 3: Fixation of hCD45+ cells in bone marrow and in peripheral blood 8 weeks after transplantation**

| | Number of mice having repopulated cells/number of transplanted mice | CD45+ cells in bone marrow of transplanted mice (%) | CD45+ cells in peripheral blood of transplanted mice (%) |
|---|---|---|---|
| Accessory cells | 0/4 | ND | ND |
| Non-coculture | 3/7 | 0.33 ± 0.47 | ND |
| Without stromal cells | 2/4 | 0.24 ± 0.30 | ND |
| Control stromal cells | 7/7 | 0.74 ± 0.53 | 0.13 ± 0.05 |
| Ihh-stromal cells | 7/7 | 10.6 ± 8.20* | 0.60 ± 0.27* |

| | | | |
|---|---|---|---|
| "ND" indicates that %hCD45 is 0.1% or smaller of the cutoff level. | | | |
| Values are represented in terms of means ± standard deviation. | | | |
| * P < 0.05 in comparison with the control stroma group (Mann-Whitney U test) | | | |

### (2) Fixation of transplanted cells (A)

The mice were sacrificed by cervical dislocation 8 or 13 weeks after the transplantation, and bone marrow and peripheral blood mononuclear cells were recovered. The presence of human hematopoietic cells was determined by detecting ALU repeats via PCR and by flow cytometry with the use of anti-human CD45-specific antibodies.

Specifically, PCR was carried out using primer sequences 5'-CACCTGTAATCCCAGCAGTTT-3' and 5'-CGCGATCTCGGCTCACTGCA-3'. DNA samples were amplified via a cycle of denaturation at 94°C for 4 minutes, denaturation at 94°C for 1 minute, annealing at 55°C for 45 seconds, and extension at 72°C for 1 minute, and this cycle was repeated 35 times. The amplified product was separated via electrophoresis on 2.5% agarose gel, stained with ethidium bromide, and then visualized as a 221-bp band.

### (Results)

Fig. 6 shows the results.

In Fig. 6A, no reaction was observed in the mice into which the accessory cells had been solely transplanted (lanes 1, 2, and 3); however, human ALU was observed in the mice into which pre-cocultured CB CD34+ cells had been transplanted (lanes 4, 5, 6, and 7).

The numerical values represent proportions of human CD45+ cells in the bone marrow of the mice that had undergone transplantation.

Fig. 6B shows the results of flow cytometric analysis, with the use of anti-human CD45 antibodies, of bone marrow mononuclear cells of the mice 8 weeks after the transplantation. Human CD45+ cells were frequently observed in the mice into which CB CD34+ cells that had been pre-cocultured with ihh-stromal cells had been transplanted. This indicates that ihh-stromal cells allow amplification of SRC in CB CD34+ cells to a greater extent than the control stromal cells.

Fig. 6C shows the results of flow cytometric analysis, with the use of anti-human CD45 antibodies, of bone marrow mononuclear cells of the mice 13 weeks after the transplantation. Even when the period after the transplantation was prolonged, human CD45+ cells were maintained in the mice into which CB CD34+ cells that had been pre-cocultured with ihh-stromal cells had been transplanted.

### (2) Examination of viability of transplanted cells

The mice were sacrificed by cervical dislocation 6 weeks after the transplantation, and bone marrow and peripheral blood mononuclear cells were recovered. The presence of human hematopoietic cells was determined by staining cells with FITC-anti-human-CD45-conjugated antibodies and analyzing the stained cells via flow cytometry.

Subsequently, flow cytometry was carried out in order to examine whether or not the cells surviving in bone marrow maintained the capacity for multipotential differentiation.

### (Results)

The results are shown in Fig. 7A and Fig. 7B.

As reported in the past (Nat Med., 1996, 2, 1329-1337), hematopoietic cells amplified with the aid of ihh can be differentiated into CD11b+, CD19+, and CD38+ cells, and the capacity for differentiation may be maintained, as with the case in which HPCs were amplified in the control stromal cells.

All publications, patents, and patent applications cited herein are incorporated herein by reference in their entirety.

### Industrial Applicability

Upon overexpression of ihh genes via introduction thereof into human bone marrow stromal cells, hematopoiesis-supporting ability can be enhanced via the direct or indirect functions of such genes.

Further, use of the ihh-expressing autologous stromal cells having a potent hematopoiesis-supporting ability obtained in the present invention enables the development of a novel technique for amplifying autologous hematopoietic stem cells.

## Claims

1. A method for proliferating hematopoietic lineage cells comprising expressing the following polypeptide (a), (b), (c), or (d) having activity of proliferating hematopoietic lineage cells using a gene encoding said polypeptide:
(a) a polypeptide as shown in SEQ ID NO: 1;
(b) a polypeptide comprising at least amino acid residues 28 to 202 in the sequence as shown in SEQ ID NO: 1;
(c) a polypeptide consisting of a sequence derived from the sequence as shown in SEQ ID NO: 1 by deletion, substitution, and/or addition of one to several amino acid residues and having activity of proliferating hematopoietic lineage cells; or
(d) a polypeptide derived from the polypeptide composed of amino acid residues 28 to 202 of the sequence as shown in SEQ ID NO: 1 by deletion, substitution, and/or addition of one to several amino acid residues and having activity of proliferating hematopoietic lineage cells.

2. A method for proliferating hematopoietic lineage cells comprising expressing the gene consisting of the following DNA (a), (b), or (c):
(a) DNA as shown in SEQ ID NO: 2;
(b) DNA comprising at least nucleotides 81 to 606 of the DNA sequence as shown in SEQ ID NO: 2; or
(c) DNA hybridizing under stringent conditions to DNA (a) or (b) and encoding a protein having Indian hedgehog activity.

3. Bone marrow stromal cells comprising the gene encoding the following polypeptide (a), (b), (c), or (d) having activity of proliferating hematopoietic lineage cells introduced therein:
(a) a polypeptide as shown in SEQ ID NO: 1;
(b) a polypeptide comprising at least amino acid residues 28 to 202 in the sequence as shown in SEQ ID NO: 1;
(c) a polypeptide consisting of a sequence derived from the sequence as shown in SEQ ID NO: 1 by deletion, substitution, and/or addition of one to several amino acid residues and having activity of proliferating hematopoietic lineage cells; or
(d) a polypeptide derived from the polypeptide composed of amino acid residues 28 to 202 of the sequence as shown in SEQ ID NO: 1 by deletion, substitution, and/or addition of one to several amino acid residues and having activity of proliferating hematopoietic lineage cells.

4. The bone marrow stromal cells according to claim 3, which are stromal cells to which human telomerase catalytic domain (hTERT) genes have been already introduced.

5. The bone marrow stromal cells according to claim 3 or 4, wherein the gene encoding the polypeptide having activity of proliferating hematopoietic lineage cells is introduced with the use of a retroviral vector.

6. A method for proliferating hematopoietic lineage cells comprising subjecting the bone marrow stromal cells according to any one of claims 3 to 5 to coculture with hematopoietic lineage cells.

7. The method according to claim 6, wherein the hematopoietic lineage cells are cord-blood-derived CD34+ cells.

8. The method according to claim 6 or 7, wherein coculture is carried out in the presence of cytokines.

9. The method according to any one of claims 6 to 8, wherein coculture is carried out for 2 to 4 weeks.

10. Artificial bone marrow comprising the bone marrow stromal cells and hematopoietic lineage cells according to any one of claims 3 to 5.

11. An expression vector for enhancing hematopoiesis comprising the gene encoding the following polypeptide (a), (b), (c), or (d) having activity of proliferating hematopoietic lineage cells:
(a) a polypeptide as shown in SEQ ID NO: 1;
(b) a polypeptide comprising at least amino acid residues 28 to 202 in the sequence as shown in SEQ ID NO: 1;
(c) a polypeptide consisting of a sequence derived from the sequence as shown in SEQ ID NO: 1 by deletion, substitution, and/or addition of one to several amino acid residues and having activity of proliferating hematopoietic lineage cells; or
(d) a polypeptide derived from the polypeptide composed of amino acid residues 28 to 202 of the sequence as shown in SEQ ID NO: 1 by deletion, substitution, and/or addition of one to several amino acid residues and having activity of proliferating hematopoietic lineage cells.

12. An agent for proliferating hematopoietic lineage cells comprising, as an active ingredient, the following polypeptide (a), (b), (c), or (d) having activity of proliferating hematopoietic lineage cells:
(a) a polypeptide as shown in SEQ ID NO: 1;
(b) a polypeptide comprising at least amino acid residues 28 to 202 in the sequence as shown in SEQ ID NO: 1;
(c) a polypeptide consisting of a sequence derived from the sequence as shown in SEQ ID NO: 1 by deletion, substitution, and/or addition of one to several amino acid residues and having activity of proliferating hematopoietic lineage cells; or
(d) a polypeptide derived from the polypeptide composed of amino acid residues 28 to 202 of the sequence as shown in SEQ ID NO: 1 by deletion, substitution, and/or addition of one to several amino acid residues and having activity of proliferating hematopoietic lineage cells.

13. The proliferating agent according to claim 12, wherein the hematopoietic lineage cells are human hematopoietic cells or human cord-blood-derived CD34+ cells.
